(19) Europäisches Patentamt
European Patent Office
Office européen des brevets

(11) Publication number: 0 130 109 B1

# (12) EUROPEAN PATENT SPECIFICATION

(45) Date of publication of patent specification: 13.09.89

(21) Application number: 84401235.1

(22) Date of filing: 15.06.84

(51) Int. Cl.[4]: C 07 D 333/34, C 07 D 333/22, C 07 D 409/12, C 07 D 409/06, A 61 K 31/38, A 61 K 9/06

## (54) Derivatives of thiophenesulfonamide, their preparation and pharmaceutical composition for the topical treatment of elevated intraocular pressure.

(30) Priority: 20.06.83 US 505673
20.06.83 US 505618
20.06.83 US 505604

(43) Date of publication of application:
02.01.85 Bulletin 85/01

(45) Publication of the grant of the patent:
13.09.89 Bulletin 89/37

(84) Designated Contracting States:
CH DE FR GB IT LI NL

(56) References cited:
EP-A-0 042 731
EP-A-0 070 239
EP-A-0 079 269
GB-A-1 459 571
GB-A-1 468 111

JOURNAL OF MEDICAL CHEMISTRY, vol. 24, no. 8, 1981, pages 959-964; J.T. BARNISH et al.: "Cerebrovasodilatation through selective inhibition of the enzyme carbonic anhydrase. 3.5-(Arylthio)-, 5-(Arylsulfinyl)-, and 5-(Arylsulfonyl)thiophene-2-sulfonamides"

(73) Proprietor: MERCK & CO. INC.
126, East Lincoln Avenue P.O. Box 2000
Rahway New Jersey 07065-0900 (US)

(72) Inventor: Shepard, Kenneth L.
136 Cardinal Way
North Wales Pennsylvania 19454 (US)

(74) Representative: Ahner, Francis et al
CABINET REGIMBEAU 26, avenue Kléber
F-75116 Paris (FR)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 130 109 B1

Courier Press, Leamington Spa, England.

## Description

Summary of the Invention

This invention relates to novel derivatives of [phenylsulfonyl(sulfinyl or carbonyl)]thiophenesulfonamides which are useful in the treatment of elevated intraocular pressure. More particularly this invention relates to derivatives having the structural formula:

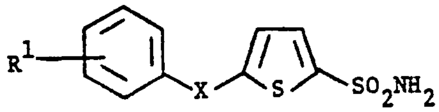

wherein X is

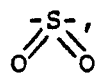 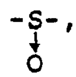 or 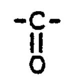 ;

and $R^1$ is as hereinafter defined, and the ophthalmologically acceptable salts thereof. This invention also relates to ophthalmic compositions that are employed in the treatment of elevated intraocular pressure, especially when accompanied by pathological damage such as in the disease known as glaucoma.

Background of the Invention

Glaucoma is an ocular disorder associated with elevated ocular pressures which are too high for normal function and may result in irreversible loss of visual function. If untreated, glaucoma may eventually lead to blindness. Ocular hypertension, i.e., the condition of elevated intraocular pressure without optic nerve head damage or characteristic glaucomatous visual field defects, is now believed by many ophthalmologists to represent the earliest phase of glaucoma.

Many of the drugs formerly used to treat glaucoma proved not entirely satisfactory. Indeed, few advances were made in the treatment of glaucoma since pilocarpine and physostigmine were introduced. Only recently have clinicians noted that many ß-adrenergic blocking agents are effective in reducing intraocular pressure. While many of these agents are effective in reducing intraocular pressure, they also have other characteristics, e.g. membrane stabilizing activity, that are not acceptable for chronic ocular use.

(S)-1-*tert*-butylamino-3-[(4-morpholino-1,2,5-thiadiazol-3-yl)oxy]-2-propanol, a ß-adrenergic blocking agent, was found to reduce intraocular pressure and to be devoid of many unwanted side effects associated with pilocarpine and, in addition, to possess advantages over many other ß-adrenergic blocking agents, e.g. to be devoid of local anaesthetic properties, to have a long duration of activity, and to display minimal tolerance.

Although pilocarpine, physostigmine and ß-blocking agents reduce intraocular pressure, none of these drugs manifests its action by inhibiting the enzyme carbonic anhydrase and, thereby, impeding the contribution made by the carbonic anhydrase pathway to aqueous humor formation.

Agents referred to as carbonic anhydrase inhibitors, block or impede this inflow pathway by inhibiting the enzyme, carbonic anhydrase. While such carbonic anhydrase inhibitors are now used to treat intraocular pressure by oral, intravenous or other systemic routes, they thereby have the distinct disadvantage of inhibiting carbonic anhydrase throughout the entire body. Such a gross disruption of a basic enzyme system is justified only during an acute attack of alarmingly elevated intraocular pressure, or when no other agent is effective. Despite the desireability of directing the carbonic anyhdrase inhibitor only to the desired ophthalmic target tissue, no topically effective carbonic anhydrase inhibitors are available for clinical use.

Detailed Description of the Invention

A preferred embodiment of the novel compounds of this invention has structural formula:

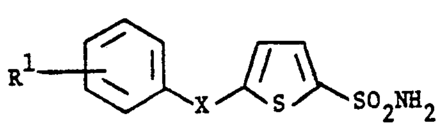

or a pharmaceutically acceptable salt thereof, wherein X is

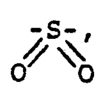 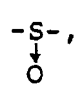 or 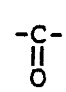 ;

$R^1$ is HO— (only when X is

$-\underset{\underset{O}{\|}}{C}-$) or $R(O)_n-\overset{\overset{O}{\|}}{C}-O-$

wherein

n is 0 or 1;

R is

1) $C_{1-18}$alkyl, either straight or branched chain,

2) $C_{1-18}$ haloalkyl, wherein halo is chloro, bromo, or fluoro,

3) $R^1$, $R^2$N-$C_{1-5}$alkyl, wherein $R^1$ and $R^2$ are independently hydrogen or $C_{1-3}$alkyl, or $R^1$ and $R^2$ are joined together to form a heterocycle selected from piperidinyl, morpholinyl, or pyrrolidinyl,

4) $C_{1-3}$alkoxycarbonyl-$C_{1-5}$alkyl,

5) $C_{3-6}$cycloalkyl,

6) $C_{3-6}$cycloalkyl-$C_{1-3}$alkyl,

7) $C_{1-3}$alkyl-$C_{3-6}$cycloalkyl,

8) aryl, wherein aryl is a carbocycle or heterocycle such as phenyl, naphthyl, pyridinyl, furanyl, thienyl, or the like, either unsubstituted or substituted with one or more of $C_{1-3}$alkyl, halo or $C_{1-3}$alkoxy,

9) aryl-$C_{1-3}$alkyl, either unsubstituted or substituted with one or more of halo, $C_{1-3}$alkyl or $C_{1-3}$alkoxy,

10) $C_{2-6}$alkenyl,

11) $C_{2-6}$alkynyl,

12) aryl-$C_{2-6}$alkenyl,

13) $NR^3$-piperidinyl, wherein $R^3$ is $C_{1-3}$alkyl or $C_{2-5}$alkanoyl, or

14) $C_{1-3}$alkoxy-$C_{1-5}$alkyl.

Representative novel compounds of this invention include those wherein the substituent group,

$$R(O)_n\overset{\overset{O}{\|}}{C}-,$$

is in the 3 or 4 position of the phenyl group and R represents: phenyl, ethyl, propyl, 1,1-dimethylethyl, heptyl, undecanyl, 4,4-dimethylcyclohexyl; 2-chloro-1,1-dimethylethyl; 4-methylphenyl; 4-chlorophenyl; 4-methoxyphenyl; 4-chlorobenzyl; 3-(4-ethylphenyl)ethyl; allyl; 2-propynyl; 3-phenylallyl; cyclopentylmethyl; benzyl; cyclohexyl; methyl; 1,1-dimethyl-2-dimethylaminoethyl; 2-(methoxycarbonyl)ethyl; 4-(1-acetylpiperidinyl); and 3-pyridyl.

Especially preferred are the compounds wherein X is

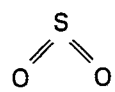

and R is $C_{1-18}$ alkyl and most particularly preferred are those compounds where R is $C_{1-5}$alkyl, either straight or branched chain. Another preferred compound is that therein $R^1$ is —OH and X is

$$-\underset{\underset{O}{\|}}{C}-$$

It is also preferred that the hydroxy or acyloxy group be in the 4-position of the phenyl.

The compounds of this invention are most suitably prepared by reacting a compound of formula;

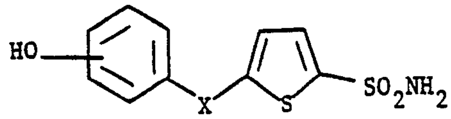

with a compound of the formula:

$$R(O)_n\overset{\overset{O}{\|}}{C}-Cl$$

or a *bis* carbonate of the formula:

$$R-O-\overset{\overset{O}{\|}}{C}-O-R$$

for those compounds wherein n = 1, or an anhydride of formula:

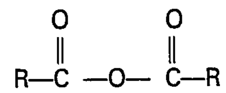

for those compounds wherein n is 0.

The reaction is conducted in a suitable solvent such as dimethylformamide, pyridine, acetone, ethyl acetate, tetrahydrofuran or benzene and the like with at least an equimolar amount of a hydrohalide acceptor. Bases such as triethylamine, pyridine and the like may be employed for this purpose.

The reaction may be conducted with or without a catalyst at temperatures of from 0°C to the boiling point of the solvent used but preferably from 15°C to 50°C.

When a catalyst is employed, triethylamine or a 4,4-dialkylaminopyridine such as 4-dimethylaminopyridine or 4-pyrrolidinopyridine is preferred.

The novel compound wherein $R^1$ is HO— is prepared by heating the compound of structure:

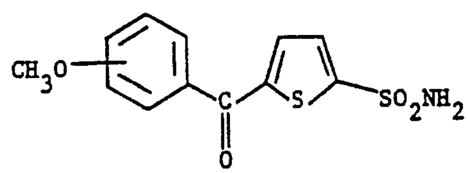

in admixture with pyridine hydrochloride between its fusion point and about 250°C, preferably at about 200°C, for about 15 minutes to about 2 hours.

The following examples describe the general preparative methods employed. Those examples that do not include an appendage describing analytical data or melting points are given as only illustrative, not having been actually conducted. They are nevertheless believed consistent with the methods described and fully workable with only the actual yield in doubt.

Example 1

4-[(2-Sulfamoyl-5-thienyl)sulfonyl]phenyl 2,2-Dimethylpropionate

A solution of 5-[(4-hydroxyphenyl)sulfonyl]-thiophene-2-sulfonamide (4.66 g, 0.01459 mole] in acetone (90 ml) at 0° was treated with triethylamine (1.62 g, 0.0160 mole) and 4-dimethylaminopyridine (75 mg). Trimethylacetic anhydride (2.98 g, 0.0160 mole) was added, dropwise, during 20 minutes at 0°. The cooling bath was removed and stirring was continued for 1 hour. The acetone was removed *in vacuo* and the residue was dissolved in ethyl acetate, washed with water and saturated sodium chloride solution and dried over sodium sulfate. The ethyl acetate was evaporated *in vacuo* to give a white solid, yield, 5.89 g; m.p. 192—195°. Recrystallization from 1,2-dichloroethane gave the title compound as white needles, m.p. 194—197°.

| | | | |
|---|---|---|---|
| Anal. Calc'd. for $C_{15}H_{17}NO_6S_3$: | C, 44.65; | H, 4.25; | N, 3.47; |
| Found: | C, 44.40; | H, 4.11; | N, 3.31. |

Example 2

4-[(2-Sulfamoyl-5-thienyl)sulfonyl]phenyl 2,2-Dimethyl-3-dimethylaminopropionate

*Step A:* Preparation of 2,2-Dimethyl-3-dimethylaminopropionyl chloride hydrochloride

A mixture of 2,2-dimethyl-3-dimethylamino-propionic acid hydrochloride (1.76 g, 0.00969 mole) and thionyl chloride [10 ml) was heated under reflux for 30 minutes.

The excess thionyl chloride was removed *in vacuo.* The residue was treated with benzene and the benzene was removed *in vacuo* to give the title compound as a white solid. The yield was 1.94 g, m.p. 191—192°.

| | | | |
|---|---|---|---|
| Anal. Calc'd. for $C_7H_{14}ClNO(+HCl)$: | C, 42.01; | H, 7.56; | N, 7.00; |
| Found: | C, 41.95; | H, 7.98; | N, 7.30. |

*Step B:* Preparation of 4-[(2-Sulfamoyl-5-thienyl)sulfonyl]phenyl 2,2-Dimethyl-3-dimethylaminopropionate

A solution of 5-[(4-hydroxyphenyl)sulfonyl]thiophene-2-sulfonamide (3.19 g, 0.01 mole) in acetone (60 ml) at −10° was treated with triethylamine (1.11 g, 0.011 mole). Then 2,2-dimethyl-3-dimethylaminopropionyl chloride hydrochloride (2.20 g, 0.011 mole) was added portionwise, during 15 minutes at −10°. The cooling bath was removed and stirring was continued. After 72 hours at room temperature, the reaction mixture was heated on a steam bath for 4 hours. The reaction mixture was cooled and treated with triethylamine (1.11 g, 0.011 mole) and heated on a steam bath for 1 hour.

After cooling to room temperature, the triethylamine hydrochloride was removed by filtration and the filtrate was evaporated *in vacuo* to give a sticky residue. Seeds were obtained by allowing a sample of the

residue to stand, at room temperature, in a mixture of ethyl acetate and isopropyl alcohol, m.p. 218—222°. Recrystallization from acetonitrile, followed by recrystallization from nitromethane gave the title compound as white needles, 1.84 g, m.p. 237—238°.

| | | | |
|---|---|---|---|
| Anal. Calc'd. for $C_{17}H_{22}N_2O_6S_3$: | C, 45.72; | H, 4.97; | N, 6.27; |
| Found: | C, 45.75; | H, 5.06; | N, 6.18. |

Example 3

4-[(2-Sulfamoyl-5-thienyl)sulfonyl]phenyl 3-(Methoxycarbonyl)propionate

A solution of 5-[(4-hydroxyphenyl)sulfonyl]thiophene-2-sulfonamide (3.19 g, 0.1 mole) in acetone (60 ml) at −10° was treated with triethylamine (1.11 g, 0.011 mole. Then methoxycarbonylpropionyl chloride (1.66 g; 0.011 mole) was added, dropwise during 5 minutes at −10°.

After 15 minutes, triethylamine hydrochloride was removed by filtration and the filtrate was evaporated *in vacuo.* The residue was dissolved in ethyl acetate, washed with water and saturated sodium chloride solution and dried over sodium sulfate. Removal of the ethyl acetate *in vacuo* gave a white solid. The yield was 4.33 g, m.p. 121—129°. Recrystallization from 1,2-dichloroethane gave the title compound as white needles, m.p. 135—136°.

| | | | |
|---|---|---|---|
| Anal. Calc'd. for $C_{15}H_{15}NO_8S_3$: | C, 41.56; | H, 3.49; | N, 3.23; |
| Found: | C, 41.71; | H, 3.47; | N, 3.43. |

Example 4

4-[(2-Sulfamoyl-5-thienyl)sulfonyl]phenyl Acetate

A solution of 5-[(4-hydroxyphenyl)sulfonyl]thiophene-2-sulfonamide (3.19 g, 0.1 mole) in acetone (60 ml) at −10° was treated with triethylamine (1.11 g, 0.011 mole). Then acetyl chloride (0.86 g, 0.011 mole) was added, dropwise during 5 minutes at −10°.

After 15 minutes, triethylamine hydrochloride was removed by filtration and the filtrate was evaporated *in vacuo.* The residue was dissolved in ethyl acetate, washed with water and saturated sodium chloride solution and dried over sodium sulfate. Removal of the ethyl acetate *in vacuo* gave a white solid. The yield was 3.61 g, m.p. 137—141°. Recrystallization from 1,2-dichloroethane gave the title compound as white prisms, m.p. 143.5—144.5°.

| | | | |
|---|---|---|---|
| Anal. Calc'd. for $C_{12}H_{11}NO_6S_3$: | C, 39.88; | H, 3.07; | N, 3.88; |
| Found: | C, 39.83; | H, 3.03; | N, 3.92. |

Example 5

4-[(2-Sulfamoyl-5-thienyl)sulfonyl]phenyl 1-acetylpiperidine-4-carboxylate

A solution of 5-[(4-hydroxyphenyl)sulfonyl]thiophene-2-sulfonamide (3.19 g, 0.1 mole) in acetone (60 ml) at −15° was treated with triethylamine (1.11 g, 0.011 mole. Then 1-acetylpiperidine-4-carbonyl chloride hydrochloride (2.41 g, 0.011 mole) was added, portionwise, during 15 minutes at −15°. The cooling bath was removed and stirring was continued. After 20 hours at room temperature, the reaction mixture was treated with triethylamine (1.11 g, 0.011 mole). Then after stirring one hour longer, the reaction mixture was treated with 1-acetylpiperidine-4-carbonyl chloride hydrochloride (2.41 g, 0.011 mole) and triethylamine (2.22 g, 0.022 mole) and stirred at room temperature.

After 30 minutes, triethylamine hydrochloride was removed by filtration and the filtrate was evaporated *in vacuo.* The residue was dissolved in ethyl acetate, washed with water and saturated sodium chloride solution and dried over sodium sulfate. Removal of the ethyl acetate *in vacuo* gave a glass-like residue. Seeds were obtained by allowing a sample of the residue to stand at room temperature in 1,2-dichloroethane, m.p. 178—185°. Recrystallization from 1,2-dichloroethane gave a white solid, 1.95 g. A second recrystallization, from acetonitrile gave the title compound, m.p. 186—189°.

| | | | |
|---|---|---|---|
| Anal. Calc'd. for $C_{18}H_{20}N_2O_7S_3$: | C, 45.75; | H, 4.27; | N, 5.93; |
| Found: | C, 45.84; | H, 4.31; | N, 6.26. |

Example 6

4-[(2-Sulfamoyl-5-thienyl)sulfonyl]phenyl Nicotinate

A solution of 5-[(4-hydroxyphenyl)sulfonyl]thiophene-2-sulfonamide (3.19 g, 0.1 mole) in dimethylformamide (30 ml) at 25° was treated with triethylamine (2.23 g, 0.022 mole). Then nicotinyl chloride hydrochloride (1.96g, 0.011 mole) was added, portionwise, during 15 minutes at 25°. Stirring was continued at room temperature.

After 16 hours, the reaction mixture was poured into water (150 ml). The resulting oil was extracted into ethyl acetate, washed with water and saturated sodium chloride solution and dried over sodium sulfate. Removal of the ethyl acetate *in vacuo* gave a sticky residue, yield 3.92 g. Recrystallization from acetonitrile gave the title compound as light yellow needles, m.p. 200—201°.

| | | | |
|---|---|---|---|
| Anal. Calc'd. for $C_{16}H_{12}N_2O_6S_3$: | C, 45.27; | H, 2.85; | N, 6.60; |
| Found: | C, 45.44; | H, 2.84; | N, 6.79. |

Employing the procedure substantially as described in Examples 1—6, but substituting for the anhydride used in Example 1 or the acid chlorides used in Examples 2—6, an equimolar amount of the reagent of formula

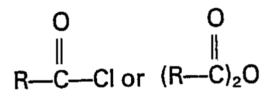

described in Table I there are prepared the acyloxy compounds also described in Table I in accordance with the following reaction:

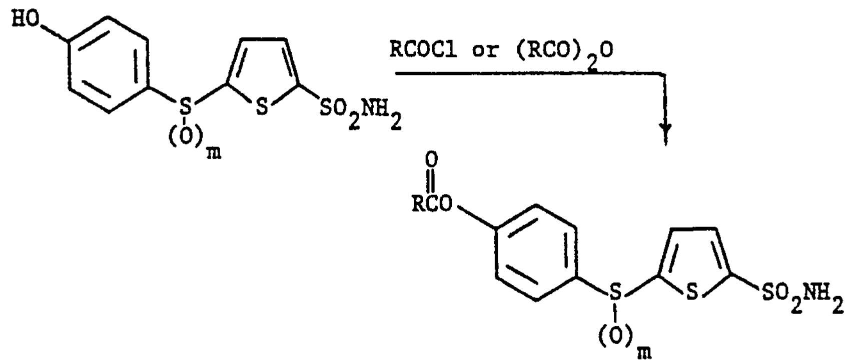


TABLE I

| R | R |
|---|---|
| phenyl, | 3-phenyl-2-propenyl, |
| ethyl, | cyclopentylmethyl, |
| propyl, | benzyl, |
| n-heptyl, | cyclohexyl, |
| n-undecanyl | 2,2-dimethylpropyl, |
| 4,4-dimethylcyclohexyl, | cinnamyl, |
| 2-chloro-1,1-dimethylethyl, | 4-pyridylmethyl, |
| 4-methylphenyl, | cyclopentyl |
| 4-chlorophenyl, | 2,2,2-trifluoroethyl, |
| 4-methoxyphenyl, | 2-propynyl, |
| 4-chlorobenzyl, | |
| 2-(4-ethylphenyl)ethyl, | |
| allyl, | |

Example 7

2-Methylpropyl-4-[(2-Sulfamoyl-5-thienyl)sulfonyl]phenyl Carbonate

A solution of 5-[(4-hydroxyphenyl)sulfonyl]thiophene-2-sulfonamide (2.50 g, 0.00783 mole) in acetone (50 ml) at 0° was treated with triethylamine (0.87 g, 0.0861 mole) followed by isobutyl chloroformate (1.17 g, 0.00861 mole) dropwise, during 30 minutes at 0—2°.

After 15 additional minutes, triethylamine hydrochloride was removed by filtation and the filtrate was evaporated *in vacuo* to give a soft white solid. After triturating with butyl chloride, the yield was 3.28 g, m.p. 103—106°. Recrystallization from butyl chloride gave the title compound as white needles, m.p. 111—112°.

Anal. Calc'd. for $C_{15}H_{17}NO_7S_3$: C, 42.95; H, 4.08; N, 3.34;
Found: C, 42.59; H, 4.05; N, 3.32.

Employing the procedure substantially described in Example 7, but substituting for the chloroformate used in Example 7 an equimolar amount of the reagent of formula

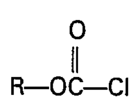

described in Table II there are prepared the carbonates also described in Table II in accordance with the following reaction:

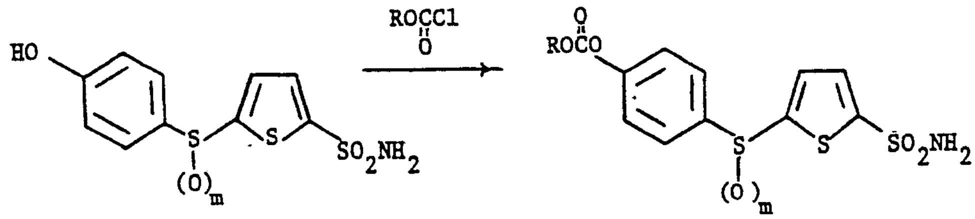


TABLE II

| R | R |
|---|---|
| phenyl, | 3-phenyl-2-propenyl, |
| propyl, | cyclopentylmethyl, |
| propyl, | benzyl, |
| n-heptyl, | cyclohexyl, |
| n-undecanyl | methyl, |
| 4,4-dimethylcyclohexyl, | 2,2-dimethylpropyl, |
| 2-chloro-1,1-dimethylethyl, | cinnamyl, |
| 4-methylphenyl, | 4-pyridylmethyl, |
| 4-chlorophenyl, | cyclopentyl |
| 4-methoxyphenyl, | 4-nitrophenyl, |
| 4-chlorobenzyl, | 2-(triphenylphosphonium)-ethyl, |
| 2-(4-ethylphenyl)ethyl, | 2,2,2-trifluoroethyl, |
| allyl, | |
| 2-propynyl, | |

Example 8

Preparation of 5-(4-Hydroxybenzoyl)thiophene-2-sulfonamide

*Step A:* Preparation of 2-Bromo-5-(4-methoxybenzoyl)thiophene

To a cold (0—5°C) solution of 2-bromothiophene (32.6 g, 0.2 mol) and p-methoxybenzoyl chloride (34.1 g, 0.2 mol) in methylene chloride was added anhydrous stannic chloride (52 g, 0.2 mol) dropwise over about one hour. When addition was complete, the cooling bath was removed and the solution was stirred for an additional two hours. A solution of water (90 ml) and concentrated hydrochloric acid (10 mL) was added dropwise and the layers were separated. The organic layer was washed with water, saturated sodium chloride solution and dried ($MgSO_4$). Removal of the solvent *in vacuo* and collection of the solid with the aid of hexane gave 48.5 g, of gray powder, m.p. 97—99°C. A sample was recrystallized from ligroin for analysis.

| | | |
|---|---|---|
| Anal. Calc'd. for $C_{12}H_9BrO_2S$: | C, 48.50; | H,3.05. |
| Found: | C, 49.11; | H, 3.00. |

*Step B:* Preparation of 2-Benzylmercapto-5-(4-methoxybenzoyl)thiophene

Benzyl mercaptan (1.24 g, 0.01 mol) was added to a stirred mixture of sodium hydride (50% oil dispersion, 0.44 g, 0.11 mol) and degassed DMP (10 mL). The resulting mixture was warmed cautiously until gas evolution ceased. After cooling to 25°C, a solution of 2-bromo-5-(4-methoxybenzoyl)thiophene (2.97 g, 0.01 mol) in DMF (10 mL) was added dropwise. After complete addition, the mixture was heated on the steam bath for 2.5 hours and poured into water (200 mL). The aqueous mixture was extracted with ether (2×200 mL) and the extracts were washed with water, saturated NaCl solution and dried ($Na_2SO_4$). After evaporation of the solvent, the residue was collected with the aid of hexane and dried, 1.98 g, m.p. 103—105°C.

| | | |
|---|---|---|
| Anal. Calc'd. for $C_{19}H_{16}O_2S_2$: | C, 67.03; | H,4.74. |
| Found: | C, 67.19; | H, 4.64. |

*Step C:* Preparation of 5-(4-Methoxybenzoyl)thiophene-2-sulfonamide

Chlorine was bubbled into a stirred cold (0—10°C) mixture of 2-benzylmercapto-5-(4-methoxybenzoyl)-thiophene (3.4 g) and 33% aqueous acetic acid (50 mL). When the temperature no longer rose during the addition (*ca.* 20 min), the pale yellow solid was filtered, washed well with water then hexane. The resulting solid was dissolved in acetone (50 mL) and added to cold (0—5°C) concentrated aqueous ammonia (100 ml). The mixture was stirred 16—24 hours (25°C) and diluted with water. The solid that separated was collected and recrystallized from 95% ethanol, m.p. 176—178°C.

| | | | |
|---|---|---|---|
| Anal. Calc'd. for $C_{12}H_{11}NO_4S_2$: | C, 48.47; | H, 3.73; | N, 4.71; |
| Found: | C, 48.20; | H, 3.70; | N, 4.74. |

*Step D:* Preparation of 5-(4-Hydroxybenzoyl)thiophene-2-sulfonamide

A mixture of 5-(4-methoxybenzoyl)thiophene-2-sulfonamide (5.48 g) and pyridine hydrochloride (50 g) was heated to 200°C for 0.5 hour. Water (400 mL) was added to the cooled reaction mixture and the solid collected by filtration. This solid was stirred with 1N hydrochloric acid (100 mL) for 0.5 hour, filtered, washed with water and dried at 70°C, 4.11 g, m.p. 183—190°C. Treatment of this material with acetonitrile, followed by sodium bicarbonate solution then 3N hydrochloric acid, and recrystallization from water gave 1.70 g, m.p. 200—201°C.

| | | | |
|---|---|---|---|
| Anal. Calc'd. for $C_{11}H_9NO_4S_2$: | C, 46.63; | H, 3.20; | N, 4.94; |
| Found: | C, 46.63; | H, 3.13; | N, 4.94. |

Example 9

4-[(2-Sulfamoyl-5-thienyl)carbonyl]phenyl 2,2-Dimethylpropionate

A solution of 5-(4-hydroxybenzoyl)thiophene-2-sulfonamide (0.01459 mole) in acetone (90 ml) at 0°C is treated with triethylamine (0.0160 mole) and 4-dimethylaminopyridine (75 mg). Trimethylacetic anhydride (0.0160 mole) is added, dropwise, during 20 minutes at 0°C. The cooling bath is removed and stirring is continued for 1 hour. The acetone is removed *in vacuo* and the residue is dissolved in ethyl acetate, washed with water and saturated sodium chloride solution and dried over sodium sulfate. The ethyl acetate is evaporated *in vacuo* to give the title compound.

Employing the procedure substantially as described in Example 9, but substituting for the anhydride used in Example 9, an equimolar amount of the reagent of formula

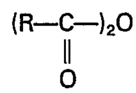

described in Table III, there are produced the acyloxy compounds also described in Table III in accordance with the following reaction:

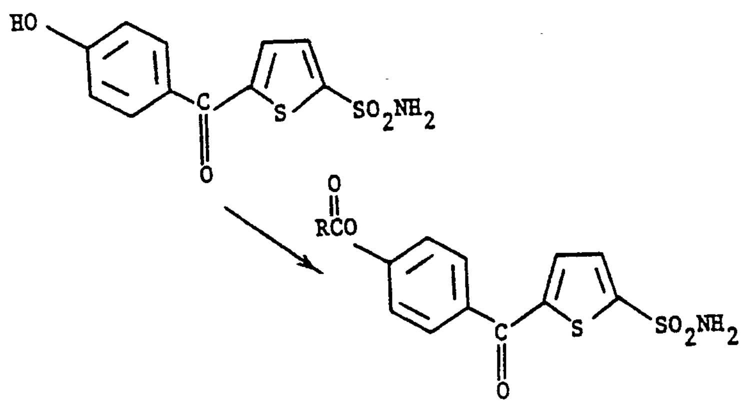

TABLE III

| R | R |
|---|---|
| phenyl, | 3-phenyl-2-propenyl, |
| ethyl, | cyclopentylmethyl, |
| propyl, | benzyl, |
| n-heptyl, | cyclohexyl, |
| n-undecanyl | 2,2-dimethylpropyl, |
| 4,4-dimethylcyclohexyl, | cinnamyl, |
| 2-chloro-1,1-dimethylethyl, | 4-pyridylmethyl, |

TABLE III (continued)

| R | R |
|---|---|
| 4-methylphenyl, | cyclopentyl |
| 4-chlorophenyl, | 2,2,2-trifluoroethyl, |
| 4-methoxyphenyl, | 2-propynyl, |
| 4-chlorobenzyl, | 1,1-dimethyl-2-dimethyl-aminoethyl, |
| 2-(4-ethylphenyl)ethyl, | 2-methoxycarbonylethyl, |
| allyl, | methyl |
| 1-acetyl-4-piperidyl, | |
| 3-pyridyl | |

Example 10

2-Methylpropyl 4-[(2-Sulfamoyl-5-thienyl)carbonyl]phenyl Carbonate

A solution of 5-(4-hydroxybenzoyl)thiophene-2-sulfonamide (0.00783 mole) in acetone (50 ml) at 0°C is treated with triethylamine (0.00861 mole) followed by isobutyl chloroformate (0.00861 mole) dropwise, during 30 minutes at 0—2°C.

After 15 additional minutes, triethylamine hdyrochloride is removed by filtration and the filtrate is evaporated *in vacuo* to give the title compound.

Employing the procedure substantially as described in Example 10, but substituting for the chloroformate used in Example 10 an equimolar amount of the reagent of formula

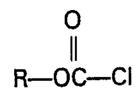

described in Table IV there are prepared the carbonates also described in Table IV in accordance with the following reaction:

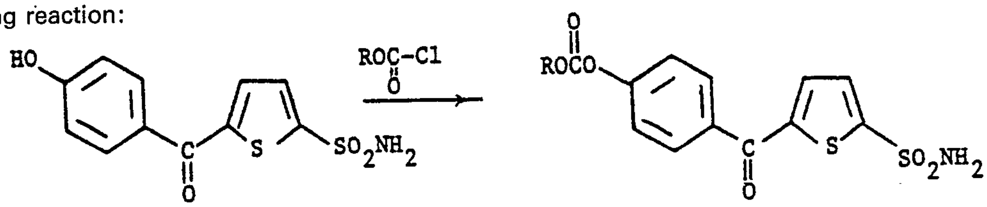

TABLE IV

| R | R |
|---|---|
| phenyl, | 3-phenyl-2-propenyl, |
| propyl, | cyclopentylmethyl, |
| propyl, | benzyl, |
| n-heptyl, | cyclohexyl, |
| n-undecanyl | methyl, |
| 4,4-dimethylcyclohexyl, | 2,2-dimethylpropyl, |
| 2-chloro-1,1-dimethylethyl, | cinnamyl, |
| 4-methylphenyl, | 4-pyridylmethyl, |
| 4-chlorophenyl, | cyclopentyl |
| 4-methoxyphenyl, | 4-nitrophenyl, |
| 4-chlorobenzyl, | 2-(triphenylphosphonium)-ethyl, |
| 2-(4-ethylphenyl)ethyl, | 2,2,2-trifluoroethyl, |
| allyl, | |
| 2-propynyl, | |

In addition to the novel compounds described above, a number of 5-[phenylsulfonyl (or sulfinyl)]-thiophene-2-sulfonamides have been described in British patent 1,459,571 and *J. Med. Chem., 24,* 959 (1981) that are active according to this invention and may be used for the manufacture of the composition for the topical treatment of glaucoma and ocular hypertension. They have structural formula:

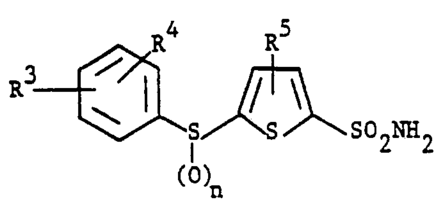

or an opthalmologically acceptable salt thereof, wherein:

n is 1 or 2;

$R^3$ is

1) —OH

$R^6$
/
2) —N wherein $R^6$ and $R^7$ are
\
$R^7$

independently selected from:

a) hydrogen,

b; $C_{1-3}$alkyl,

c) $C_{2-5}$alkanoyl,

d) $C_{3-6}$cycloalkylcarbonyl,

e) benzoyl,

f) $C_{1-3}$alkoxycarbonyl,

g) N-$C_{1-3}$alkyl carbamoyl,

h) $C_{1-3}$alkylsulfonyl,

$R^8$
/
3) —$SO_2N$ wherein $R^8$ and $R^9$ are
\
$R^9$

independently selected from hydrogen, $C_{1-3}$alkyl or $C_{3-6}$cycloalkyl,

4) —$CH_2CO_2H$

$R^8$
/
3) —CON
\
$R^9$

6) —$CO_2H$, or $R^8$

$R^8$
/
3) —$CH_2CON$
\
$R^9$

$R^4$ is

1) hydrogen,

2) halo, such as chloro, bromo or fluoro,

3) $C_{1-3}$alkyl or

4) $C_{1-3}$alkoxy; and

$R^5$ is

1) hydrogen, or

2) halo, such as chloro or bromo.

A preferred embodiment of the compounds active in the novel compositions of this invention includes those compounds wherein:

n is 2;

$R^3$ is

1) —OH,

$R^6$
/
2) —N wherein $R^6$ and $R^7$ are independently selected from;
\
$R^7$

a) hydrogen,

b) $C_{2-5}$alkanoyl,

c) $C_{1-3}$alkoxysulfonyl,

d) $C_{1-3}$alkoxycarbonyl,

e) N-$C_{1-3}$alkylcarbamoyl,

f) $C_{3-6}$cycloalkylcarbonyl,

and $R^4$ and $R^5$ are both hydrogen.

Representative species are those defined by the following table wherein n is 1 or 2;

| | $R^3$ | $R^4$ | $R^5$ |
|---|---|---|---|
| 1) | 4-$NHCOCH_3$ | H | H |
| 2) | 4-$NHCOC_6H_5$ | H | H |
| 3) | 4-$SO_2NH_2$ | H | H |
| 4) | 4-$NHCOC_2H_5$ | H | H |
| 5) | 4-$NHCOCH_3$ | 2-Cl, 5-$CH_3$ | H |
| 6) | 4-$NHSO_2CH_3$ | H | H |
| 7) | 4-$NHCOOCH_3$ | H | H |
| 8) | 3-$NHCOCH_3$ | 4-$OCH_3$ | H |
| 9) | 4-$CH_2COOH$ | H | H |
| 10) | 4-$CH_2CONHCH_3$ | H | H |
| 11) | 4-$NHCONHCH_3$ | H | H |
| 12) | 4-$NHCOCH_3$ | 3-Cl | H |
| 13) | 4-$NHCOCH_3$ | H | 4-Br |
| 14) | 4-$NHCOCH_3$ | H | 3-Cl |
| 15) | 4-$NHCOCH(CH_3)_2$ | H | H |
| 16) | 4-$NHCOCH_2CH_2CH_3$ | H | H |
| 17 | 4-$NHCOC(CH_3)_3$ | H | H |
| 18) | 4-$NHCOCH_3$ | H | 4-Cl |
| 19) | 4-$NHCOCH_3$ | H | 3-Br |
| 20) | 3-$NHCOOC_2H_5$ | H | H |
| 21) | 4-NHCO 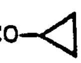 | H | H |
| 22) | 4-$CONH_2$ | H | H |
| 23) | 3-$CO_2H$ | H | H |
| 24) | 3-$CONH_2$ | H | H |
| 25) | 3-$CONHCH_3$ | H | H |
| 26) | 3-$CONHCH(CH_3)_2$ | H | H |
| 27) | 4-$CH_2CONH_2$ | H | H |
| 28) | 2-$CONH_2$ | H | H |
| 29) | 3-$NHCOCH_3$ | H | H |
| 30) | 4-OH | H | H |
| 31) | 4-$CH_2CONHC(CH_3)_3$ | H | H |
| 32) | 3-CONH 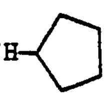 | H | H |

The most preferred species is 5-[(4-hydroxyphenyl)sulfonyl]thiophene-2-sulfonamide.

For use in treatment of conditions relieved by the inhibition of carbonic anhydraze, the active compound is administered topically in the treatment of the eye. The dose administered can be from as little as 0.1 to 25 mg or more per day, singly, or preferably on a 2 to 4 dose per day regimen although a single dose is satisfactory.

When administered for the treatment of elevated intraocular pressure or glaucoma, the active compound is administered topically to the eye.

When given by the topical route, the active drug or an ophthalmologically acceptable salt thereof such as the sodium or potassium salt is formulated into an ophthalmic preparation.

In such formulations, from 0.1% to 15% by weight can be employed. The objection is to administer a dose of from 0.1 to 10 mg per eye per day to the patient, with treatment continuing so long as the condition persists.

Thus, in an ophthalmic solution, insert, ointment or suspension for topical delivery, the active medicament or an equivalent amount of a salt thereof is employed, the remainder being carrier, excipients, preservatives and the like as are customarily used in such compositions.

In the form of an ophthalmic solution, the active drug can be employed as ophthalmologically acceptable salts such as the sodium and potassium salts obtained by neutralizing an equivalent of the sulfonamide with an equivalent of a suitable base such as, for example, an alkali metal hydroxide.

The active drug of this invention is most suitably administered in the form of ophthalmic pharmaceutical compositions adapted for topical administration to the eye such as a suspension, ointment, or as a solid insert. Formulations of these compounds may contain from 0.01 to 15% and especially 0.5% to 2% of medicament. Higher dosages as, for example, about 10%, or lower dosages can be employed provided the dose is effective in reducing or controlling elevated intraocular pressure. As a unit dosage from beteen 0.001 to 10.0 mg, preferably 0.005 to 2.0 mg, and espcially 0.1 to 1.0 mg of the compound is generally applied to the human eye, generally on a daily basis in single or divided doses so long as the condition being treated exists.

These hereinbefore described dosage values are believed accurate for human patients and are based on the known and presently understood pharmacology of the compounds, and the action of other similar entities in the human eye. They reflect the best mode known. As with all medications, dosage requirements are variable and must be individualized on the basis of the disease and the response of the patient.

The pharmaceutical preparation which contains the active compound may be conveniently admixed with a non-toxic pharmaceutical organic carrier, or with a non-toxic pharmaceutical inorganic carrier. Typical of pharmaceutically acceptable carriers are, for example, water, mixtures of water and water-miscible solvents such as lower alkanols or aralkanols, vegetable oils, polyalkylene glycols, petroleum based jelly, ethyl cellulose, ethyl oleate, carboxymethylcellulose, polyvinylpyrrolidone, isopropyl myristate and other conventionally employed acceptable carriers. The pharmaceutical preparation may also contain non-toxic auxiliary substances such as emulsifying, preserving, wetting agents, bodying agents and the like, as for example, polyethylene glycols 200, 300, 400 and 600, carbowaxes 1,000, 1,500, 4,000, 6,000 and 10,000, antibacterial components such as quaternary ammonium compounds, phenylmercuric salts known to have cold sterilizing properties and which are non-injurious in use, thimerosal, methyl and propyl paraben, benzyl alcohol, phenylethanol, buffering ingredients such as sodium chloride, sodium borate, sodium acetates, gluconate buffers, and other conventional ingredients such as sorbitan monolaurate, triethanolamine, oleate, polyoxythylene sorbitan monoplamitylate, dioctyl sodium sulfosuccinate, monothioglycerol, thiosorbitol, ethylenediamine tetracetic acid, and the like. Additionally, suitable ophthalmic vehicles can be used as carrier media for the present purpose including conventional phosphate buffer vehicle systems, isotonic boric acid vehicles, isotonic sodium chloride vehicles, isotonic sodium borate vehicles and the like. The pharmaceutical preparation may also be in the form of a solid insert.

While many patients find liquid medication to be entirely satisfactory, others may prefer a solid medicament that is topically applied to the eye, for example, a solid dosage form that is suitable for insertion into the cul-de-sac. To this end the carbonic anhydrase inhibiting agent can be included with a non-bioerodible insert, i.e. one which after dispensing the drug remains essentially intact, or a bioerodible insert, i.e. one that either is soluble in lacrimal fluids, or otherwise disintegrates. While the insert employed is not critical and those disclosed in 3,630,200 Higuchi; 3,811,444 Heller et al.; 4,177,256 Michaels et al.; 3,868,445 Ryde et al; 3,845,201 Haddad; 3,981,303 Higuchi; and 3,867,519 Michaels, are satisfactory; in general, however, the insert described below is found preferable.

For example, one may use a solid water soluble polymer as the carrier for the medicament. The polymer used to form the insert may be any water soluble non-toxic polymer, for example, cellulose derivatives such as methylcellulose, sodium carboxymethyl cellulose, or a hydroxyl lower alkyl cellulose such as hydroxyethyl cellulose, hydroxypropyl cellulose, hydroxypropylmethyl cellulose and the like; acrylates such as polyacrylic acid salts, ethyl acrylates, polyacrylamides; natural products such as gelatin, alginates, pectins tragacanth, karaya, chondrus, agar, acacia; the starch derivatives such as starch acetate, hydroxyethyl starch ethers, hydroxypropyl starch, as well as other synthetic derivatives such as polyvinyl alcohol, polyvinyl pyrrolidone, polyvinyl methyl ether, polyethylene oxide, neutralized carbopol and xanthan gum, and mixtures of said polymer.

Preferably the solid insert is prepared from cellulose derivatives such as methylcellulose, hydroxyethyl cellulose, hydroxypropyl cellulose or hydroxypropylmethyl cellulose or from other synthetic materials such as polyvinyl alcohol, polyvinyl pyrrolidone, polyethylene oxide or polyvinyl methylether. Hydroxypropyl cellulose, one of the preferred polymers for the preparation of the insert, is available in several polymeric forms, all of which are suitable in the preparation of these inserts. Thus, the product sold

by Hercules, Inc. of Wilmington, Delaware, under the name KLUCEL such as KLUCEL HF, HWF, MF, GF, JF, LF and EF which are intended for food or pharmaceutical, use, are particularly useful. The molecular weight of these polymers useful for the purposes described herein may be at least 30,000 to about 1,000,000 or more.

Example 11

| | |
|---|---|
| 4-[(2-Sulfamoyl-5-thienyl)sulfonyl]phenyl Nicotinate (I) | 5 mg. |
| petrolatum q.s. ad. | 1 gram |

Compound I and the petrolatum are aseptically combined.

Example 12

| | |
|---|---|
| 4-[(2-Sulfamoyl-5-thienyl)sulfonyl]phenyl Acetate | 1 mg. |
| Hydroxypropylcellulose q.s. | 12 mg. |

Ophthalimic inserts are manufactured from compression molded films which are prepared on a Carver Press by subjecting the powdered mixture of the above ingredients to a compressional force of 12,000 lbs. (gauge) at 300°F for one to four minutes. The film is cooled under pressure by having cold water circulate in the platen. Ophthalmic inserts are then individually cut from the film with a rod-shaped punch. Each insert is placed into a vial, which is then placed in a humidity cabinet (88% R.H. at 30°C) for two to four days. After removal from the humidity cabinet, the vials are stoppered and then capped. The vials containing the hydrate insert are then autoclaved at 250°F for ½ hour.

Example 13

| | |
|---|---|
| 4-[(2-Sulfamoyl-5-thienyl)sulfonyl]phenyl 2,2-Dimethylpropionate | 1 mg. |
| Hydroxypropyl cellulose q.s. ad. | 12 mg. |

Ophthalimic inserts are manufactured from a solvent cast film prepared by making a viscous solution of the powdered ingredients listed above using methanol as the solvent. The solution is placed on a Teflon plate and allowed to dry at ambient conditions. After drying, the film is placed in an 88% R.H. cabinet until it is pliable. Appropriately sized inserts are cut from the film.

Example 14

| | |
|---|---|
| 4-[(2-Sulfamoyl-5-thienyl)sulfonyl]phenyl 2,2-Dimethyl-3-dimethylaminopropionate | 1 mg. |
| Hydroxypropyl methyl cellulose q.s. ad. | 12 mg. |

Ophthalmic inserts are manufactured from a solvent cast film which is prepared by making a viscous solution of the powdered blend of the above ingredients using a methanol/water solvent system (10 ml. methanol is added to 2.5 g. of the powdered blend, to which 11 ml. of water (in three divided portions) is added. The solution is placed on a Teflon plate and allowed to dry at ambient conditions. After drying, the film is placed in an 88% R.H. cabinet until it is pliable. Appropriately sized inserts are then cut from the film.

Example 15

| | |
|---|---|
| 4-[(2-Sulfamoyl-5-thienyl)sulfonyl]phenyl 3-(Methoxycarbonyl)propionate | 1 mg. |
| Hydroxypropylmethyl cellulose q.s. ad. | 12 mg. |

Ophthalimic inserts are manufactured from compression molded films which are prepared on a Carver Press by subjecting the powdered mixture of the above ingredients to a compressional force of 12,000 lbs. (gauge) at 350°F for one minute. The film is cooled under pressure by having cold water circulate in the platen. Ophthalimic inserts are then individually cut from the film with a punch. Each insert is placed into a vial, which is then placed in a humidity cabinet (88% R.H. at 30°C) for two to four days. After removal from the humidity cabinet, the vials are stoppered and then capped. The vials containing the hydrated insert are

then autoclaved at 250°F for one-half hour.

It is highly preferred that the solid inserts of this invention are available for use by the patient in a pathogen free condition. Thus, it is preferred to sterilize the inserts and so as insure against recontamination, the sterilization is preferably conducted after packaging. The best mode of sterilizing is to employ ionizing irradiation including irradiation emanating from Cobalt 60 or high energy electron beams.

After packaging a convenient quantity of inserts, usually a single dose, the package is exposed to a sterilizing quantity of radiation. The preferred packaging employs sealing the inserts between layers of film or foil and then sealing or laminating the layers together about the edges. The techniques for performing the sterilization are well known and accepted, for example, as outlined in International Atomic Energy Commission, *Code of Practice for Radiosterilization of Medical Products*, 1967, pp. 423—431; and Block, *Disinfection, Sterilization and Preservation*, 2nd Ed., Lea & Febiger, Philadelphia, 1977, pp. 542—561.

The required quantity of irradiation can be determined experimentally by testing irradiated inserts for viable bacteria. Generally, the amount of irradiation desired to achieve sterilization is defined by the $D_{10}$ value. The $D_{10}$ value is the radiation dose that will reduce a given population of organisms by a factor of 10. Based on $D_{10}$ values, experimentally obtained for *Bacillus pumilus*, and presterilization contamination levels, a dose of 1.36 megarads is effective in obtaining a sterile product.

**Claims**

1. A compound of structural formula:

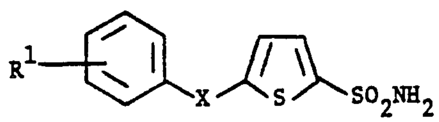

or a pharmaceutically acceptable salt thereof, wherein

X is

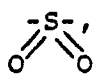

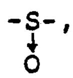

or

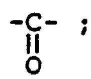

$R^1$ is HO— (only when X is

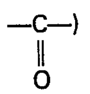

or

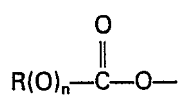

n is 0 or 1;

R is

1) $C_{1-18}$alkyl, either straight or branched chain,

2) $C_{1-18}$ haloalkyl, wherein halo is chloro, bromo, or fluoro,

3) $R^1$, $R^2N$-$C_{1-5}$alkyl, wherein $R^1$ and $R^2$ are independently hydrogen or $C_{1-3}$alkyl, or $R^1$ and $R^2$ are joined together to form a heterocycle selected from piperidinyl, morpholinyl, or pyrrolidinyl,

4) $C_{1-3}$alkoxycarbonyl-$C_{1-5}$alkyl,

5) $C_{3-6}$cycloalkyl,

6) $C_{3-6}$cycloalkyl-$C_{1-3}$alkyl,

7) $C_{1-3}$alkyl-$C_{3-6}$cycloalkyl,

8) aryl, wherein aryl is a carbocycle or heterocycle selected from phenyl, naphthyl, pyridinyl, furanyl, thienyl, or the like, either unsubstituted or substituted with one or more of $C_{1-3}$alkyl, halo or $C_{1-3}$alkoxy,

9) aryl-$C_{1-3}$alkyl, either unsubstituted or substituted with one or more of halo, $C_{1-3}$alkyl or $C_{1-3}$alkoxy,

10) $C_{2-6}$alkenyl,

11) $C_{2-6}$alkynyl,

12) aryl-$C_{2-6}$alkenyl,

13) $NR^3$-piperidinyl, wherein $R^3$ is $C_{1-3}$alkyl or $C_{2-5}$alkanoyl, or

14) $C_{1-3}$alkoxy-$C_{1-5}$alkyl.

2. The compound according to Claim 1 wherein X is

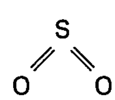

and R is $C_{1-18}$ alkyl and is part of a 4-substituent.

3. The compound of Claim 1, wherein $R^1$ is HO— and X is

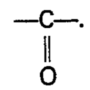

4. An ophthalmic composition for the topical treatment of glaucoma and ocular hypertension comprising an intraocular pressure lowering effective amount of the compound of claim 1 and an ophthalmologically acceptable carrier.

5. The compound of Claim 4 wherein X is

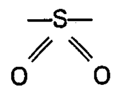

and R of the carbonic anhydrase inhibitor is $C_{1-18}$ alkyl and is part of a 4-substituent.

6. The composition of Claim 1, wherein R is HO— and X is

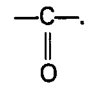

7. A process for preparing the compound of Claim 1 of the formula:

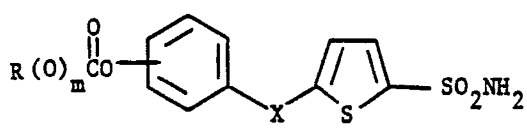

comprising reacting a compound of the formula:

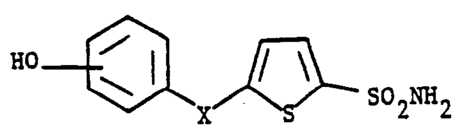

with a compound of the formula:

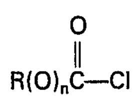

or a carbonate of formula:

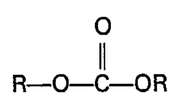

wherein n=1, or an anhydride of formula:

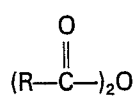

wherein n=0.

8. The process of Claim 7 where X is

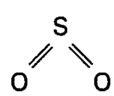

R is $C_{1-18}$alkyl and is part of a 4-substituent.

9. Use for the manufacture of the composition for the topical treatment of glaucoma and ocular hypertension, of a compound having the formula

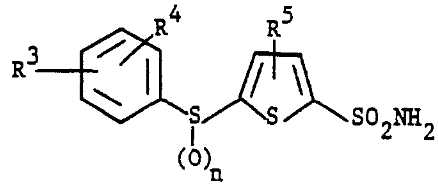

or an ophthalmologically acceptable salt thereof, wherein:

n is 1 or 2;

$R^3$ is 1) —OH

2) 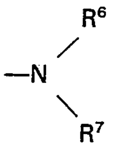

wherein $R^6$ and $R^7$ are independently:

a) hydrogen,
b) $C_{1-3}$alkyl,
c) $C_{2-5}$alkanoyl,
d) $C_{3-6}$cycloalkylcarbonyl,
e) benzoyl,
f) $C_{1-3}$alkoxycarbonyl,
g) N—$C_{1-3}$alkyl carbamoyl, or
h) $C_{1-3}$alkylsulfonyl,

3) 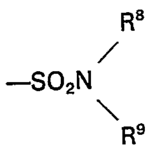

wherein $R^8$ and $R^9$ are independently:

a) hydrogen,
b) $C_{1-3}$alkyl, or
c) $C_{3-6}$cycloalkyl,

4) —$CH_2CO_2H$,

5) 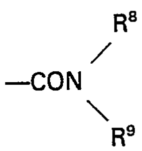

6) —$CO_2H$, or

7) 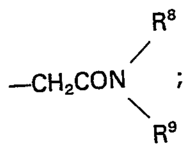 ;

$R^4$ is 1) hydrogen,
2) halo,
3) $C_{1-3}$ alkyl, or
4) $C_{1-3}$ alkoxy; and

$R^5$ is 1) hydrogen, or
2) halo.

10. The composition of Claim 9 wherein:

$R^3$ is 1) —OH

2) 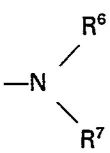

wherein

$R^6$ and $R^7$ are independently:

a) hydrogen,
b) $C_{2-5}$alkanoyl,
c) $C_{1-3}$alkylsulfonyl,
d) $C_{1-3}$alkoxycarbonyl,
e) N—$C_{1-3}$alkyl carbamoyl,
f) $C_{3-6}$cycloalkylcarbonyl; and

$R^4$ and $R^5$ are both hydrogen.

## Patentansprüche

1. Verbindung der Strukturformel

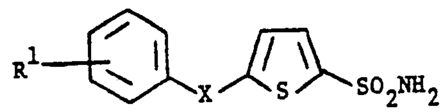

oder ein pharmazeutisch annehmbares Salz davon, worin

X

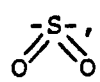 , 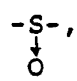 , oder 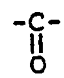 ;

ist;

$R^1$ HO—

(nur, wenn X $\underset{\underset{O}{\|}}{-C-}$ ist) oder $R(O)_n-\overset{\overset{O}{\|}}{C}-O-$ ist;

n 0 oder 1 ist;

R 1) $C_{1-18}$-Alkyl, entweder geradkettig oder verzweigt,
2) $C_{1-18}$-Halogenalkyl, worin Halogen Chlor, Brom oder Fluor ist,
3) $R^1$, $R^2$N—$C_{1-5}$-Alkyl, worin $R^1$ und $R^2$ unabhängig Wasserstoff oder $C_{1-3}$-Alkyl sind oder $R^1$ und R unter Bildung eines aus Piperidinyl, Morpholinyl oder Pyrrolidinyl ausgewählten Heterocyclus zusammengenommen werden,
4) $C_{1-3}$-Alkoxycarbonyl-$C_{1-5}$-alkyl,
5) $C_{3-6}$-Cycloalkyl,
6) $C_{3-6}$-Cycloalkyl-$C_{1-3}$-alkyl,
7) $C_{1-3}$-Alkyl-$C_{3-6}$-cycloalkyl,
8) Aryl, worin Aryl ein Carbocyclus oder Heterocyclus ist, ausgewählt aus Phenyl, Naphthyl, Pyridinyl, Furanyl, Thienyl oder dergleichen, entweder unsubstituiert oder mit einem oder mehreren aus $C_{1-3}$-Alkyl, Halogen oder $C_{1-3}$-Alkoxy substituiert,
9) Aryl-$C_{1-3}$-alkyl, entweder ubsubstituiert oder mit einem oder mehreren Halogen, $C_{1-3}$-Alkyl oder $C_{1-3}$-Alkoxy substituiert,
10) $C_{2-6}$-Alkenyl,
11) $C_{2-6}$-Alkinyl,
12) Aryl-$C_{2-6}$-alkenyl,
13) $NR^3$-Piperidinyl, worin $R^3$ $C_{1-3}$-Alkyl oder $C_{2-5}$-Alkanoyl ist, oder
14) $C_{1-3}$-Alkoxy-$C_{1-5}$alkyl ist.

2. Verbindung nach Anspruch 1, worin X

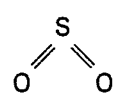

ist und R $C_1$—$C_{18}$-Alkyl und Teil eines 4-Substituenten ist.

3. Verbindung nach Anspruch 1, worin $R^1$ HO— ist und X

$\underset{\underset{O}{\|}}{-C-}$ ist.

4. Ophthalmische Zusammensetzung zur topischen Behandlung von Glaukom und Augenhochdruck, welche eine den Augeninnendruck vermindernde wirksame Menge der Verbindung nach Anspruch 1 und einen ophthalmologisch annehmbaren Träger umfaßt.

5. Zusammensetzung nach Anspruch 4, worin X

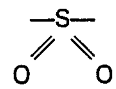

ist und R des kohlenstoffhaltigen Anhydraseinhibitors $C_1$—$C_{18}$-Aklyl und Teil eines 4-Substituenten ist.

6. Zusammensetzung nach Anspruch 1, worin R HO— ist und X

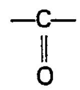 ist.

7. Verfahren zur Herstellung der Verbindung nach Ansporuch 1 der Formel

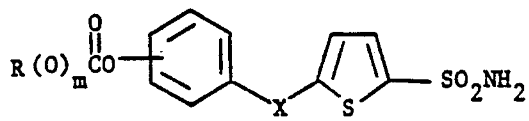

durch Reagieren einer Verbindung der Formel

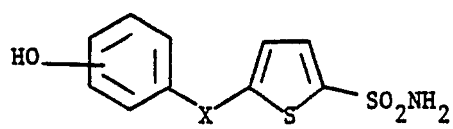

mit einer Verbindung der Formel:

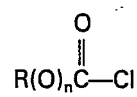

oder einem Carbonat der Formel

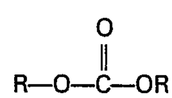

worin n=1, oder einem Anhydrid der Formel:

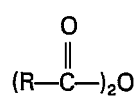

worin n=0.

8. Verfahren nach Anspruch 7, worin X

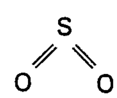

ist und R $C_1$—$C_{18}$-Alkyl und Teil eines 4-Substituenten ist.

9. Verwendung zur Herstellung der Zusammensetzung für die topische Behandlung von Glaukom und Augenhochdruck einer Verbindung mit der Formel

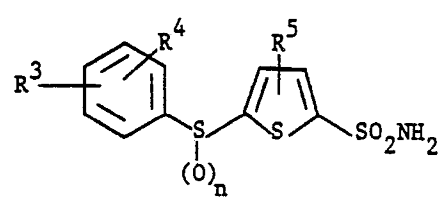

oder eines ophthalmologisch annehmbaren Salzes davon, worin:

n 1 oder 2 ist;

$R^3$ 1) —OH

2) 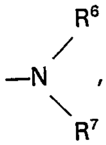 ,

worin $R^6$ und $R^7$ unabhängig:

a) Wasserstoff,
b) $C_{1-3}$-Alkyl,
c) $C_{2-5}$-Alkanoyl,
d) $C_{3-6}$-Cycloalkylcarbonyl,
e) Benzoyl,
f) $C_{1-3}$-Alkoxycarbonyl,
g) N—$C_{1-3}$-Alkylcarbamoyl, oder
h) $C_{1-3}$-Alkylsulfonyl sind,

3) 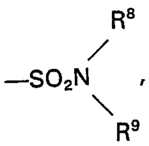 ,

worin $R^8$ und $R^9$ unabhängig

a) Waserstoff,
b) $C_{1-3}$-Alkyl, oder
c) $C_{3-6}$-Cycloalkyl sind,

4) —$CH_2CO_2H$,

5) 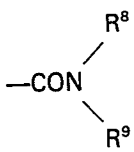

6) —$CO_2H$, oder

7) 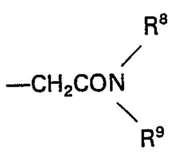 ist;

$R^4$ 1) Wasserstoff,
2) Halogen,
3) $C_{1-3}$-Alkyl oder
4) $C_{1-3}$-Alkoxy; und

$R^5$ 1) Wasserstoff oder
2) Halogen ist.

10. Zusammensetzung nach Anspruch 9, worin

$R^3$ 1) —OH

2) 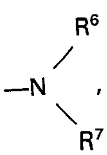 ,

worin

$R^6$ und $R^7$ unahbängig

a) Wasserstoff,
b) $C_{2-5}$-Alkanoyl,
c) $C_{1-3}$-Alkylsulfonyl,
d) $C_{1-3}$-Alkoxycarbonyl,

e) N—$C_{1-3}$-Alkylcarbamoyl,
f) $C_{3-6}$-Cycloalkylcarbonyl sind, ist und
$R^4$ and $R^5$ beide Wasserstoff sind.

## Revendications

1. Composé de formule développée:

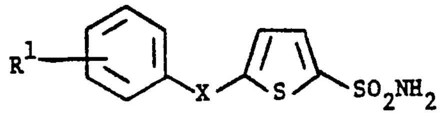

ou un sel convenant en pharmacie de celui-ci, dans laquelle X est

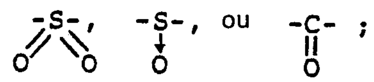

ou ;

$R^1$ est OH—
(uniquement lorsque X est

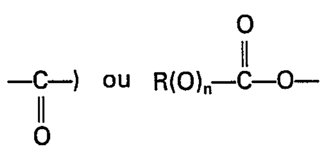

ou

où
n vaut 0 ou 1;
R est 1) un alkyle en $C_{1-18}$ à chaîne droite ou ramifiée,
2) un halogénoalkyl en $C_{1-18}$ dont l'halogéno est chloro, bromo ou fluoro,
3) $R^1$, $R^2$N-alkyl en $C_{1-5}$ où $R^1$ et $R^2$ sont indépendamment un hydrogène ou un alkyle en $C_{1-3}$, ou $R^1$ et $R^2$ sont réunis pour former un hétérocycle choisi parmi pipéridyle, morpholinyle ou pyrrolidinyle,
4) un (alcoxy en $C_{1-3}$)carbonyl-alkyle en $C_{1-5}$,
5) un cycloalkyl en $C_{3-6}$,
6) un (cycloalkyl en $C_{3-6}$)alkyle en $C_{1-3}$,
7) un (alkyl en $C_{1-3}$)cycloalkyle en $C_{3-6}$,
8) un aryle, cet aryle étant un carbocycle ou un hétérocycle, choisi parmi phényle, naphthyle, pyridyle, furyle, thiényle ou similaires, soit non substitué soit sibstitué par un ou plusieurs alkyles en $C_{1-3}$, halogéno ou alcoxy en $C_{1-3}$,
9) un aryl-alkyle en $C_{1-3}$ soit non substitué soit substitué par un ou plusieurs halogéno, alkyles en $C_{1-3}$ ou alcoxy en $C_{1-3}$,
10) un alcényle en $C_{2-6}$,
11) un alcynyle en $C_{2-6}$,
12) un aryle-alcényle en $C_{2-6}$,
13) un $NR^3$-pipéridyle, où $R^3$ est un alkyle en $C_{1-3}$ ou un alcanoyle en $C_{2-5}$, ou
14) un (alcoxy en $C_{1-3}$)alkyle en $C_{1-5}$.

2. Composé selon la revendication 1, dans lequel X est

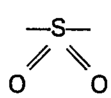

et R est un alkyle en $C_{1-18}$ et fait partie d'un substituant en position 4.

3. Composé selon la revendication 1, où $R^1$ est OH— et X est

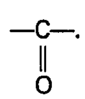

4. Composition ophtalmique pour le traitement local du glaucome et de l'hypertension oculaire comprenant une quantité efficace pour abraisser la pression intro-oculaire, d'un composé de la revendication 1 et d'un support convenant en ophtalmaloaie.

5. Composition selon la revendication 4, dans laquelle X est

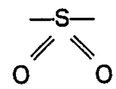

et R de l'inhibiteur de l'anhydrase carbonique est un alkyle en $C_{1-}$.507514 et fait partie d'un substituant en position 4.

6. Composition selon la revendication 1, dans laquelle R est HO— et X est

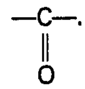

7. Procédé pour préparer un composé selon la revendication 1 de formule:

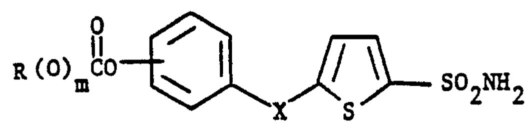

comprenant la réaction d'un composé de formule:

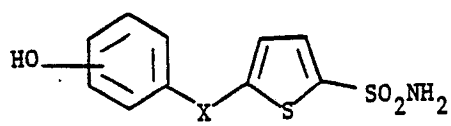

avec un composé de formule:

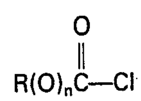

ou un carbonate de formule:

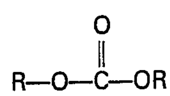

dans laquelle n = 1, ou un anhydride de formule:

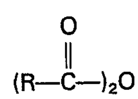

dans laquelle n = 0.

8. Procédé selon la revendication 7, dans lequel X est

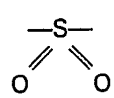

et R est un alkyle en $C_{1-18}$ et fait partie d'un substituant en position 4.

9. Emploi, pour la fabrication d'une composition pour le traitement local du glaucome et de l'hypertension oculaire, d'un composé de formule

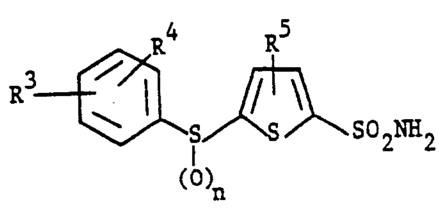

ou d'un sel convenant en ophtalmologie de celui-ci, où:

n vaut 1 ou 2;

$R^3$ est

1) —OH

2) 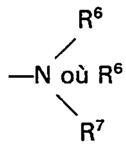 où $R^6$ et $R^7$ sont indépendamment:

a) un hydrogène,
b) un alkyle en $C_{1-3}$,
c) un alcanoyle en $C_{2-5}$,
d) un (cycloalkyl en $C_{3-6}$)carbonyle,
e) un benzoyle,
f) un (alcoxy en $C_{1-3}$)carbonyle,
g) un N-(alkyl en $C_{1-3}$)carbamoyle, ou
h) un alkylsulfonyle en $C_{1-3}$,

3) 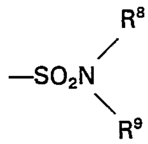

dans laquelle $R^8$ et $R^9$ sont indépendamment:

a) un hydrogène,
b) un alkyle en $C_{1-3}$, ou
c) un cycloalkyle en $C_{3-6}$,

4) $—CH_2CO_2H$,

5) 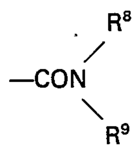

6) $—CO_2H$, ou

7) 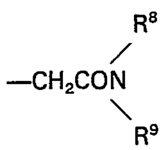

$R^4$ est 1) un hydrogène,
2) un halogéno,
3) un alkoxy en $C_{1-3}$; et
4) un alcoxy en $C_{1-3}$; et

$R^5$ est 1) un hydrogène ou
2) un halogéno.

10. Composition selon la revendication 9, dans laquelle:

$R^3$ est 1) —OH,

2) 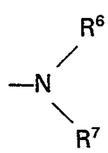

dans laquelle $R^6$ et $R^7$ sont indépendamment choisis parmi:

a) un hydrogène,
b) un alcanoyle en $C_{2-5}$,
c) un (alcoxy en $C_{1-3}$)sulfonyle,
d) un (alcoxy en $C_{1-3}$)carbonyle,
e) un N-(alkyl en $C_{1-3}$)carbamoyle,
f) un (cycloalkyl en $C_{3-6}$)carbonyle,

et $R^4$ et $R^5$ sont tous deux un hydrogène.